(19) Europäisches Patentamt
European Patent Office
Office européen des brevets

(11) Veröffentlichungsnummer: **0 023 676**
**B1**

(12) # EUROPÄISCHE PATENTSCHRIFT

(45) Veröffentlichungstag der Patentschrift: **27.07.83**

(51) Int. Cl.³: **A 61 K 7/06**, A 61 K 7/32

(21) Anmeldenummer: **80104361.3**

(22) Anmeldetag: **24.07.80**

(54) **Wirkstoffkombination für kosmetische Mittel.**

(30) Priorität: **02.08.79 DE 2931379**

(43) Veröffentlichungstag der Anmeldung: **11.02.81 Patentblatt 81/6**

(45) Bekanntmachung des Hinweises auf die Patenterteilung: **27.07.83 Patentblatt 83/30**

(84) Benannte Vertragsstaaten: **AT BE CH DE FR GB IT LI LU NL**

(56) Entgegenhaltungen:
**DE-B-1 236 729**
**FR-A-2 191 904**
**GB-A-2 031 942**
**US-A-3 236 733**
**US-A-3 281 366**
**US-A-3 678 156**
**FETTE, SEIFEN, ANSTRICHMITTEL, Band 82, Mai 1980, Seiten 177–216, Leinfelden-Echterdingen, DE.**

(73) Patentinhaber: **REWO Chemische Werke GmbH, Industriegebiet West, D-6497 Steinau an der Strasse (DE)**

(72) Erfinder: **Melloh, Wilhelm, Dr.rer.nat., Bergstrasse 7, D-6483 Bad Soden-Salm (DE)**
Erfinder: **Tanck, Robert, Kurfürstenstrasse 30, D-4000 Düsseldorf 1 (DE)**

(74) Vertreter: **Wallis, Martin, Dr. Dipl.-Chem., Waldstrasse 14, D-4619 Bergkamen (DE)**

**EP 0 023 676 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Wirkstoffkombination für kosmetische Mittel

Die Erfindung betrifft eine Wirkstoffkombination für kosmetische Mittel mit synergistischer Wirkung von zwei an sich einzeln bekannten kosmetischen Wirkstoffen.

Aus der DE-A-1 236 729 sind fungizide und bakterizide Mittel bekannt, die als Wirkstoff Undecylensäurealkylolamid-Sulfobernsteinsäurehalbester enthalten.

In der US-A-3 236 733 ist ein Verfahren zum Bekämpfen von Schuppen mit Zubereitungen beschrieben, die Metallsalze von Pyridinthionen, wie Zink-di-1-oxypyridinthion, enthalten. Pyridinthion ist eine synonyme Bezeichnung von 2-Mercaptopyridin-N-oxid.

In der JP-AS Sho-46-29 155 ist ein schorfbeseitigendes Haarpflegemittel beschrieben, das einen Gehalt einer Kombination von 3-Trifluormethyl-4,4'-dichlorcarboanilid, 3,4,4'-Trichlorcarboanilid und Undecylensäuremonoäthanolamidsulfobernsteinsäureester-Natriumsalz enthält.

Die DE-A-2 262 375 zeigt Haarbehandlungsmittel mit Antischuppenwirkung, die eine lösliche Kombination von Zink- oder Zirkonpyridinthion enthalten.

In der FR-A-2 191 904 werden kosmetische Zubereitungen als Antischuppenmittel beschrieben, die 1-Hydroxy-2-pyridone als Wirkstoff enthalten und als Beispiele für anionische waschaktive Substanzen u. a. Sulfobernsteinsäurehalbester und Diester nennen. Wegen der Zielrichtung auf 1-Hydroxy-2-pyridone führt diese Patentschrift von der vorliegenden Erfindung weg.

Das 2-Mercaptopyridin-N-oxid und dessen Derivate, wie Metallsalze und Dimere, sind zwar sehr wirksame bakterizide und fungizide Mittel, doch ist ihre Toxizität relativ hoch und auch ihre Hautverträglichkeit ist nicht befriedigend. Die letale Dosis (LD 50) von beispielsweise Zink-di-oxypyridinthion beträgt für Mäuse 300 mg/kg Körpergewicht und für Ratten 180/200 mg/kg Körpergewicht bei oraler Verabreichung in 24 Stunden. Die Verbindung ist daher zum Beispiel gemäß dem Schweizerischen Giftgesetz in Klasse 3 einzuordnen.

Aufgabe der Erfindung ist es deshalb, eine Wirkstoffkombination zur Verfügung zu stellen, die nur mit einem geringen Anteil an 2-Mercaptopyridin-N-oxid auskommt, wobei die fungizide und Antischuppen-Wirkung dieses Wirkstoffes durch einen synergistisch wirkenden Zusatz erhöht wird.

Erfindungsgemäß wird diese Aufgabe gelöst durch eine Wirkstoffkombination aus bestimmten Undecylensäurealkanolamidosulfobernsteinsäurehalbestersalzen und 2-Mercaptopyridin-N-oxid, bestimmten Salzen davon oder seinen über eine Disulfidbrücke gebildeten Dimeren.

Die Wirkstoffkombination ist von einer überraschend guten fungiziden und Antischuppen-Wirksamkeit bei niedriger Toxizität und guter Hautverträglichkeit. Sie eignet sich in erster Linie als Zusatz für Haarpflegemittel, wie Haarwaschmittel und Haarwässer, oder Desodorantien und ähnliche Körperpflegemittel. Überraschenderweise zeigt die Kombination der beiden einzeln bekannten Wirkstoffe eine unerwartet gute Wirksamkeit, die höher ist als aus der Addition der Wirksamkeit der Einzelstoffe zu erwarten war. Ein weiterer Vorteil der neuen Wirkstoffkombination ist außer der niedrigen Toxizität, der besseren Hautverträglichkeit und ihrer guten Wirksamkeit darin zu sehen, daß sie zu niedrigeren Kosten zu Verfügung steht als vergleichbare Mittel, die nur 2-Mercapto-pyridin-N-oxid oder ein Derivat davon als Wirkstoff enthalten.

Als Undecylensäurealkanolamido-Sulfobernsteinsäurehalbester verwendet man bevorzugt denjenigen, der sich vom Monoäthanolamid der Undecylensäure ableitet. Als Alkanolamidkomponente kommen jedoch auch andere Alkanolamine in Betracht, die bevorzugt nicht mehr als 8 Kohlenstoffatome im Molekül enthalten, wie zum Beispiel Isopropanolamin, Propanolamin und Butanolamine. In der Regel liegen die Undecylensäurealkanolamido-Sulfobernsteinsäurehalbester in Form ihrer Alkali- oder Ammoniumsalze vor.

Als Derivate des 2-Mercaptopyridin-N-oxids sind seine Salze und das über eine Disulfidbrücke gebundene Dimere bevorzugt. Beispiele von geeigneten Salzen sind Alkalisalze, wie das Natrium- und Kalisalz, Erdalkalisalze, wie das Magnesiumsalz, und Salze anderer zweiwertiger Metalle, wie das Zink-, Cadmium-, Zinn- und Zirkonsalz.

Bei einer besonders bevorzugten Wirkstoffkombination nach der Erfindung ist das Derivat des 2-Mercaptopyridin-N-oxids sein Zinksalz.

In der erfindungsgemäßen Wirkstoffkombination ist in der Regel der Undecylensäurealkanolamido-Sulfobernsteinsäurehalbester der überwiegende Gewichtsanteil. Bevorzugt enthält die Wirkstoffkombination 50 bis 95 Gew.-%, insbesondere 80 bis 90 Gew.-% Undecylensäurealkanolamido-Sulfobernsteinsäurehalbester und 5 bis 50 Gew.-%, insbesondere 10 bis 20 Gew.-% 2-Mercaptopyridin-N-oxid oder sein Derivat.

Die geringere Toxizität der Wirkstoffkombination gegenüber einer Alleinverwendung von 2-Mercaptopyridin-N-oxid und seiner Derivate wird dadurch erreicht, daß deren Einsatzkonzentration erheblich gesenkt werden kann. Dies kommt auch einer Forderung des Europarats entgegen, der in einer 1978 herausgegebenen Studie die Begrenzung der Einsatzkonzentration von beispielsweise Natriumpyridinthion auf 0,5% fordert. Ferner wird durch die Kosmetikverordnung in der Fassung vom 16. 12. 1977 durch das Deutsche Bundesgesundheitsamt eine Einschränkung der Konzentration an Zinksalzen des Pyridinthions gefordert bei einer Höchstkonzentration von 1%, berechnet als Zink. Der Undecylensäurealkanolamido-Sulfobernsteinsäurehalbester ist vollkommen untoxisch. Die LD 50 für

Mäuse bei oraler Applikation liegt über 10 000 mg/kg Körpergewicht.

Der Anteil der Wirkstoffkombination in dem einzelnen kosmetischen Mittel schwankt in Abhängigkeit von dem Anwendungsgebiet des speziellen kosmetischen Mittels. Haarwaschmittel mit Antischuppenwirkung enthalten in der Regel 2 bis 6 Gew.-% der Wirkstoffkombination, bezogen auf das Gesamtgewicht des Haarwaschmittels. Bei Haarwässern empfiehlt es sich, die Wirkstoffkombination in Mengen von 1 bis 5% des Gewichts, bezogen auf das Gesamtgewicht des Haarwassers, zu verwenden. Für desodorierende Mittel eignet sich die neue Wirkstoffkombination besonders in Mengen von 1 bis 6 Gew.-%, bezogen auf das Gesamtgewicht des desodorierenden Mittels.

In der Regel wird die Wirkstoffkombination in wäßrigen oder wasserhaltigen Zubereitungen verwendet. Neben der Wirkstoffkombination kann das kosmetische Mittel übliche Zusatzstoffe enthalten, die in ihrer Zusammensetzung in Abhängigkeit von dem Anwendungsgebiet schwanken.

Derartige Zusatzstoffe sind in der Hauptsache anionische und/oder nicht-ionogene und/oder amphotere Tenside. Daneben werden rückfettende Substanzen, Pflanzenextrakte, Parfümöle, Farbstoffe und Konservierungsmittel zugesetzt. Als rückfettende Substanzen kommen übliche Zusatzstoffe, wie Fettsäurealkylolamide, Proteinhydrolysate oder Lanolin und dessen Derivate in Frage. Ein solches Haarwaschmittel auf Basis anionischer Tenside hat beispielsweise folgende Zusammensetzung:

| | | |
|---|---|---|
| Natriumlauryläthersulfat, | 28%ig (in Wasser) | 50 Teile |
| Natriumlaurylsulfat, | 28%ig (in Wasser) | 10 Teile |
| Stearinsäuremonoglycerid | | 4 Teile |
| Kokosfettsäurediäthanolamid | | 2 Teile |
| Natriumchlorid | | 2 Teile |
| Wasser, Parfüm, Farbe, Konservierungsmittel | ad | 100 Teile |

Durch Hinzufügen der erfindungsgemäßen Wirkstoffkombination erhält dieses Haarwaschmittel eine Antischuppenwirkung.

Für einen klinischen Gebrauchstest wurden zum Vergleich zwei **Haarshampoos obiger** Zusammensetzung unter Zusatz folgender Wirkstoffkombination eingesetzt:

| | | |
|---|---|---|
| Haarshampoo Nr. 1: | 1% | Zink-1-oxy-2-pyridinthion |
| Haarshampoo Nr. 2: | 0,4% | Zink-1-oxy-2-pyridinthion |
| | 2,5% | Undecylensäuremonoäthanolamidosulfosuccinat |

Das Haarshampoo Nr. 2 enthält infolgedessen eine bevorzugte Wirkstoffkombination nach der Erfindung, bei der die einzelnen Wirkstoffkomponenten folgende Formeln haben

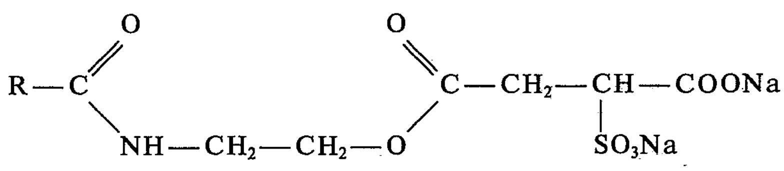

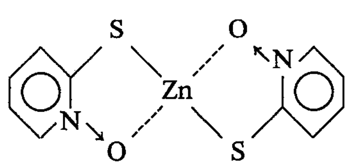

Haarshampoo Nr. 1 dient zum Vergleich.

Bei dem klinischen Gebrauchstest wurden 50 Testpersonen zunächst 3 Wochen mit Haarshampoo Nr. 1 behandelt und anschließend mit Haarshampoo Nr. 2. Parallel dazu wurden weitere 50 Testpersonen zunächst 3 Wochen mit Haarshampoo Nr. 2 behandelt und danach 3 Wochen mit Haarshampoo Nr. 1. Die Ergebnisse dieses Versuches sind in Tabelle 1 dargestellt:

Tabelle I

| Bewertung | Zahl der Testpersonen nach | | | |
|---|---|---|---|---|
| | 1.–3. Woche Nr. 1 Shampoo | 4.–6. Woche Nr. 2 Shampoo | 1.–3. Woche Nr. 2 Shampoo | 4.–6. Woche Nr. 1 Shampoo |
| Überdurchschnittlich besser | 3 | 26 | 4 | 7 |
| Weniger Schuppen | 23 | 6 | 23 | 4 |
| Etwas weniger Schuppen | 6 | 6 | 9 | 1 |
| Keine Wirkung | 13 | 9 | 11 | 30 |
| Schlechter als vor der Behandlung | 2 | 0 | 0 | 5 |

Diese Beurteilung zeigt, daß die schuppenvermindernde Wirkung in beiden Fällen, das heißt sowohl bei der Anwendung vor als auch nach derjenigen des Shampoos 1 für das synergistische Gemisch eindeutig günstiger ausfiel.

Die nachfolgenden Beispiele erläutern die Erfindung noch näher. Alle Angaben über Teile und Prozente sind Gewichtsangaben, falls nicht ausdrücklich etwas anderes festgestellt wird.

Beispiel 1

Antischuppenshampoo auf der Basis von anionischen Tensiden

| | |
|---|---|
| Dinatriumfettalkoholpolyglykoläther-sulfosuccinat (40%ig wäßrig) | 20,0 Teile |
| Triäthanolammoniumlaurylsulfat (30%ig wäßrig) | 30,0 Teile |
| Zn-di-1-oxy-2-pyridinthion | 0,8 Teile |
| Undecylensäuremonoäthanolamido-Sulfobernsteinsäurehalbester (50%ig wäßrig) | 5,0 Teile |
| Polyäthylenglykoldistearat | 2,0 Teile |
| Kokosfettsäurediäthanolamid | 3,0 Teile |
| Glykoldistearat | 1,5 Teile |
| Zitronensäure | 0,2 Teile |
| Ammoniumchlorid | 2,5 Teile |
| Wasser | 35,0 Teile |
| | 100,0 Teile |

Zur Herstellung werden alle Bestandteile in einem heizbaren Rührkessel gemischt und auf 60 bis 70° C erhitzt. Das Produkt wird homogen trüb und erhält beim Abkühlen einen stabilen Perlglanz.

Beispiel 2

Haarshampoo auf der Basis amphoterer Tenside mit besonders gutem Aufziehvermögen

| | |
|---|---|
| N-$\beta$-Hydroxyäthyl-N-carboxymethyl-Fettsäureamidoäthylamin (50%ig wäßrig) | 25,0 Teile |
| Triäthanolammoniumlaurylsulfat (40%ig wäßrig) | 20,0 Teile |
| Undecylensäuremonoäthanolamidosulfosuccinat (50%ig wäßrig) | 6,0 Teile |
| Zink-di-1-oxy-2-pyridinthion | 0,5 Teile |
| Mg-Al-Silikat | 3,5 Teile |
| Linolsäurediäthanolamid | 2,0 Teile |
| Wasser, Parfüm, Farbe, Konservierungsmittel | 43,0 Teile |
| | 100,0 Teile |

Die Herstellung dieses Shampoos erfolgt durch intensives Mischen bei 50 bis 60°C. Das Mg-Al-Silikat dient der Solubilisierung des Zink-di-1-oxy-2-pyridinthions, das für sich nicht wasserlöslich ist.

Beispiel 3

Haarwasser mit Antischuppenwirkung

| | |
|---|---|
| Isopropylalkohol | 40,0 Teile |
| n-Propylalkohol | 10,0 Teile |
| Undecylensäurediäthanolamid | 5,0 Teile |
| Undecylensäuremonoäthanolamidosulfo-succinat (50%ig wäßrig) | 2,0 Teile |
| Natriumoxy-2-pyridinthion | 0,2 Teile |
| Glycerin | 4,0 Teile |
| Diisopropyladipat | 2,0 Teile |
| Parfümöl | 0,8 Teile |
| Wasser | 36,0 Teile |
| | 100,0 Teile |

Zur Herstellung werden die wasserlöslichen Anteile und die alkohollöslichen Anteile zunächst getrennt vorgemischt und die fertigen Lösungen bei Raumtemperatur zusammengegeben und bis zur klaren Lösung gerührt.

Beispiel 4

Desodorierende Creme (Grundformulierung)

| | |
|---|---|
| Stearinsäure | 12,0 Teile |
| Cetylalkohol | 5,0 Teile |
| Stearinsäurepolyglykolester | 6,0 Teile |
| Sorbitlösung (50%ig wäßrig) | 13,0 Teile |
| Undecylensäuremonoäthanolamidosulfosuccinat (50%ig wäßrig) | 6,0 Teile |
| Dipyridin-N-oxiddisulfid | 0,2 Teile |
| Wasser, Parfüm, Konservierungsmittel | 57,8 Teile |
| | 100,0 Teile |

Zur Herstellung werden die Bestandteile zusammen bei ca. 75°C homogenisiert und langsam kalt gerührt.

**Patentansprüche**

1. Wirkstoffkombination für kosmetische Mittel, dadurch gekennzeichnet, daß sie aus

A) 50—95 Gewichts-% eines Undecylensäurealkanolamidosulfobernsteinsäurehalbestersalzes, bei dem die Alkanolamido-Komponente 2—8 Kohlenstoffatome aufweist und das Salz in Form eines Alkalimetall- oder Ammoniumsalzes vorliegt,
B) 5—50 Gewichts-% 2-Mercaptopyridin-N-oxid, seinen Alkali-, Erdalkalimetallsalzen sowie Zink-, Cadmium-, Zinn- und Zirkonsalzen oder seinem über eine Disulfidbrücke gebildeten Dimeren besteht.

2. Wirkstoffkombination für kosmetische Mittel nach Anspruch 1, dadurch gekennzeichnet, daß sie aus

A) 80—90 Gewichts-% eines Undecylensäurealkanolamidosulfobernsteinsäurehalbestersalzes, bei dem die Alkanolamido-Komponente 2—8 Kohlenstoffatome aufweist und das Salz in Form eines Alkalimetall- oder Ammoniumsalzes vorliegt,
B) 10—20 Gewichts-% 2-Mercaptopyridion-N-oxid, seinen Alkali-, Erdalkalimetallsalzen sowie Zink-, Cadmium-, Zinn- und Zirkonsalzen oder seinem über eine Disulfidbrücke gebildeten Dimeren besteht.

3. Wirkstoffkombination für kosmetische Mittel nach Anspruch 1 und 2, dadurch gekennzeichnet, daß die Alkanolamido-Komponente 2—4 C-Atome aufweist.

4. Haarwaschmittel mit Antischuppenwirkung, dadurch gekennzeichnet, daß es 2—6 Gewichts-% der Wirkstoffkombination nach Anspruch 1--3, bezogen auf das Gesamtgewicht des Haarwaschmittels, enthält.

5. Haarwasser mit Antischuppenwirkung, dadurch gekennzeichnet, daß es 1—5 Gewichts-% der Wirkstoffkombination nach Anspruch 1—3, bezogen auf das Gesamtgewicht des Haarwassers, enthält.

6. Deodorant, dadurch gekennzeichnet, daß es 1—6 Gewichts-% der Wirkstoffkombination nach Anspruch 1—3, bezogen auf das Gesamtgewicht des Deodorants, enthält.

**Claims**

1. Combination of active ingredients for cosmetic agents, characterised in that it comprises

A) from 50 to 95% by weight of a semi-ester salt of undecylenic acid alkanolamidosulphosuccinic acid in which the alkanolamido component has from 2 to 8 carbon atoms and the salt is in the form of an alkali metal salt or an ammonium salt and

B) from 5 to 50% by weight of 2-mercaptopyridine-N-oxide, the alkali metal salts, alkaline earth metal salts and zinc, cadmium, tin and zirconium salts thereof, or the dimer thereof formed via a disulphide bridge.

2. Combination of active ingredients for cosmetic agents according to claim 1, characterised in that it comprises

A) from 80 to 90% by weight of a semi-ester salt of undecylenic acid alkanolamidosulphosuccinic acid in which the alkanolamido component has from 2 to 8 carbon atoms and the salt is in the form of an alkali metal salt or an ammonium salt and

B) from 10 to 20% by weight of 2-mercaptopyridine-N-oxide, the alkali metal salts, alkaline earth metal salts and zinc, cadmium, tin and zirconium salts thereof, or the dimer thereof formed via a disulphide bridge.

3. Combination of active ingredients for cosmetic agents according to claims 1 and 2, characterised in that the alkanolamido component has from 2 to 4 carbon atoms.

4. Shampoo having an anti-dandruff action, characterised in that it contains from 2 to 6% by weight of the combination of active ingredients according to claims 1 to 3, based on the total weight of the shampoo.

5. Hair lotion having an anti-dandruff action, characterised in that it contains from 1 to 5% by weight of the combination of active ingredients according to claims 1 to 3, based on the total weight of the hair lotion.

6. Deodorant, characterised in that it contains from 1 to 6% by weight of the combination of active ingredients according to claims 1 to 3, based on the total weight of the deodorant.

**Revendications**

1. Association de matières actives pour des produits cosmétiques, association caractérisée en ce qu'elle est constituée:

A) de 50 à 95% en poids d'un sel d'hémiester sulfosuccinique d'alcanolamide de l'acide undécénoïque dont la composante alcanolamide contient de 2 à 8 atomes de carbone, le sel étant un sel de métal alcalin ou d'ammonium, et

B) de 5 à 50% en poids de mercapto-2 pyridine-N-oxyde, de ses sels de métaux alcalins ou de métaux alcalinoterreux ainsi que de ses sels de zinc, de cadmium, d'étain et de zirconium ou de son dimère formé au moyen d'un pont disulfure.

2. Association de matières actives pour des produits cosmétiques selon la revendication 1, association caractérisée en ce qu'elle est constituée:

A) de 80 à 90% en poids d'un sel d'hémiester sulfosuccinique d'alcanolamide de l'acide undécénoïque dont la composante alcanolamide contient de 2 à 8 atomes de carbone, le sel étant un sel de métal alcalin ou d'ammonium, et

B) de 10 à 20% en poids de mercapto-2 pyridine-N-oxyde, de ses sels de métaux alcalins ou de métaux alcalinoterreux ainsi que de ses sels de zinc, de cadmium, d'étain et de zirconium ou de

son dimère formé au moyen d'un pont disulfure.

3. Association de matières actives pour des produits cosmétiques selon l'une des revendications 1 et 2, association caractérisée en ce que la composante alcanolamide contient de 2 à 4 atomes de carbone.

4. Shampooing à action antipelliculaire, caractérisé en ce qu'il contient de 2 à 6% en poids d'une association de matières actives selon l'une quelconque des revendications 1 à 3, par rapport au poids total du shampooing.

5. Lotion capillaire à action antipelliculaire, caractérisée en ce qu'elle contient de 1 à 5% en poids d'une association de matières actives selon l'une quelconque des revendications 1 à 3, par rapport au poids total de la lotion capillaire.

6. Produit désodorisant caractérisé en ce qu'il contient de 1 à 6% en poids d'une association de matières actives selon l'une quelconque des revendications 1 à 3, par rapport au poids total du produit désodorisant.